(19) Europäisches Patentamt / European Patent Office / Office européen des brevets

(11) **EP 4 403 079 A1**

(12) **EUROPEAN PATENT APPLICATION**
published in accordance with Art. 153(4) EPC

(43) Date of publication:
**24.07.2024 Bulletin 2024/30**

(51) International Patent Classification (IPC):
**A47K 10/16** (2006.01)

(21) Application number: **21957296.3**

(86) International application number:
**PCT/CN2021/130870**

(22) Date of filing: **16.11.2021**

(87) International publication number:
**WO 2023/040031 (23.03.2023 Gazette 2023/12)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.09.2021 CN 202111076257**

(71) Applicants:
• **Beijing Bloomage Hyingc and Technology Co., Ltd**
  **Beijing 101407 (CN)**

• **Bloomage Biotechnology Corporation Limited**
  **Jinan, Shandong 250101 (CN)**

(72) Inventors:
• **LIU, Zhe**
  **Jinan, Shandong 250101 (CN)**
• **YANG, Juan**
  **Jinan, Shandong 250101 (CN)**

(74) Representative: **Abel & Imray LLP**
  **Westpoint Building**
  **James Street West**
  **Bath BA1 2DA (GB)**

(54) **TISSUE CONTAINING SODIUM HYALURONATE AND PREPARATION METHOD THEREFOR**

(57)  Disclosed are a tissue containing sodium hyaluronate and a preparation method therefor. The average fiber length of the tissue is 0.7-1.4 mm; the water content in the tissue is 8-20 wt%; the water activity A of the tissue satisfies the following formula: $A < -13.78 * M^2 + 4.89 * M + 0.3$, wherein M is the water content in the tissue. The tissue contains sodium hyaluronate and a water activity modulator; by adjusting the relationship between the water activity and moisture in the tissue, the friction force of the high-water-content tissue on which sodium hyaluronate is coated can be significantly reduced.

EP 4 403 079 A1

**EP 4 403 079 A1**

**Description**

**FIELD OF THE INVENTION**

The present application relates to the technical field of paper product preparation, and in particular to a tissue containing sodium hyaluronate and a preparation method thereof.

**BACKGROUND OF THE INVENTION**

Sodium hyaluronate is a natural skin moisturizer. Coating it on tissues can improve the moisturizing of the tissues to the skin. However, the strong water absorption of sodium hyaluronate makes the water content of the coated tissues significantly higher than that of ordinary tissues. For example, the water content of tissues coated with sodium hyaluronate is usually 8 to 20%, while the water content of ordinary tissues is usually 5 to 7%.
The increase in the water content of tissues will cause the cohesion between fibers to decrease. Therefore, when the tissues rub against the skin, the fibers on the interface will form a chaotic arrangement, increasing the friction and causing the stratum corneum of the skin to wear. This greatly reduces the overall moisturizing effect of the tissue.

**SUMMARY OF THE INVENTION**

In view of the above problems, the present application provides a tissue containing sodium hyaluronate. The tissue contains sodium hyaluronate and a water activity regulator, which can reduce the friction of the tissue.
The significance of the present application lies that one or more components that can form stronger hydrogen bonds with water are introduced into the tissue, thereby reducing the interaction between moisture and fibers. This component is called a water activity regulator. By adjusting the relationship between the water activity coefficient and water content in tissues, the friction of high-water-content tissues coated with sodium hyaluronate can be significantly reduced.
The specific technical solutions of the present application are as follows:

1. A tissue containing sodium hyaluronate with an average fiber length of 0.7-1.4mm and a water content of 8-20wt%, wherein the water activity A of the tissue conforms to the following formula:

$$A < -13.78*M^2 + 4.89*M + 0.3,$$

wherein M is the water content in the tissue.
2. The tissue according to item 1, wherein the tissue contains sodium hyaluronate and a water activity regulator, preferably, based on 1 part by weight of sodium hyaluronate, the water activity regulator is 40-1600 parts by weight.
3. The tissue according to item 2, wherein the molecular weight of the sodium hyaluronate is 100-2,500 kDa.
4. The tissue according to item 2 or 3, wherein the water activity regulator is a substance containing a hydroxyl group, a carboxyl group or an aldehyde group, preferably glycerol, sucrose, glucose, fructose, polyethylene glycol, butylene glycol, pentylene glycol, mannitol, sorbitol, polysorbate, or combinations thereof.
5. The tissue according to any one of items 1 to 4, wherein the content of the sodium hyaluronate in the tissue is 40-5700 ppm.
6. The tissue according to any one of items 1 to 5, wherein the cellulose content of the tissue is not less than 50% of the total mass of the tissue.
7. The tissue according to any one of items 1 to 6, wherein the tissue further contains essential oils or essences;

preferably, the tissue further contains skin care ingredients;
Preferably, the grammage of the tissue is 10-200gsm.

8. A tissue finishing liquid, comprising hyaluronic acid and a water activity regulator; preferably, based on 1 part by weight of sodium hyaluronate, the water activity regulator is 40-1600 parts by weight;

preferably, the molecular weight of the sodium hyaluronate is 100-2,500 kDa;
preferably, the water activity regulator is a substance containing a hydroxyl group, a carboxyl group or an aldehyde group, preferably glycerol, sucrose, glucose, fructose, polyethylene glycol, butylene glycol, pentylene glycol, mannitol, sorbitol, polysorbate, or combinations thereof.

9. A method for preparing a tissue, wherein the issue is obtained by spraying a solution containing hyaluronic acid and a water activity regulator on a tissue base material.

10. The method according to item 9, wherein the molecular weight of sodium hyaluronate is 100-2,500 kDa;

preferably, the content of the sodium hyaluronate in the tissue is 40-5700ppm;

preferably, the water activity regulator is a substance containing a hydroxyl group, a carboxyl group or an aldehyde group, preferably glycerol, sucrose, glucose, fructose, polyethylene glycol, butylene glycol, pentylene glycol, mannitol, sorbitol, polysorbate, or combinations thereof;

preferably, based on 1 part by weight of sodium hyaluronate, the water activity regulator is 40-1600 parts by weight.

## EFFECT OF THE INVENTION

The tissue of the present application contains sodium hyaluronate and a water activity regulator. By adjusting the relationship between the degree of the water activity and moisture in the tissue, the friction of high-water-content tissues coated with sodium hyaluronate can be significantly reduced.

## BRIEF DESCRIPTION OF THE DRAWINGS

Fig. 1 is a schematic diagram of the friction testing method in Experimental Example 3.

Fig. 2A is a schematic diagram of the fiber arrangement of a tissue containing hyaluronic acid in its original state in case 2.

Fig. 2B is a schematic diagram of the fiber arrangement of a tissue containing hyaluronic acid rubbed once at a pressure of 150Pa in case 2.

Fig. 3A is a schematic diagram of the fiber arrangement of the tissue in its original state in case 9.

Fig. 3B is a schematic diagram of the fiber arrangement of a tissue containing hyaluronic acid rubbed once at a pressure of 150Pa in case 9.

Fig. 4A is a schematic diagram of the fiber arrangement of the tissue in its original state in case 13.

Fig. 4B is a schematic diagram of the fiber arrangement of a tissue containing hyaluronic acid rubbed once at a pressure of 150Pa in case 13.

## DETAIL DESCRIPTION OF THE INVENTION

The present application will be described in detail below with reference to the embodiments described in the accompanying drawings, wherein the same numerals in all the drawings represent the same features. Although specific Examples of the present application are shown in the drawings, it should be understood that the present application may be embodied in various forms and should not be limited to the Examples set forth herein. Rather, these Examples are provided for thorough understanding of the present application, and to fully convey the scope of present application to those skilled in the art.

It should be noted that certain words are used in the description and claims to refer to specific components. Those skilled in the art will understand that skilled persons may use different nouns to refer to the same component. This description and the claims do not use difference in nouns as a way to distinguish components, but rather use differences in functions of the components as a criterion for distinction. If the words "comprise" or "include" mentioned throughout the description and claims are open-ended terms, they should be interpreted as "including but not limited to". The following descriptions of the description are preferred embodiments for implementing the present application. However, the descriptions are for the purpose of general principles of the description and are not intended to limit the scope of the present application. The protection scope of the present application shall be determined by the appended claims.

Mechanistically speaking, there are two types of cohesive forces in tissues: (1) the cohesive force between fibers; (2) the hydrogen bonding effect between moisture and fibers. If the hydrogen bonding effect is too strong, the cohesion between fibers will be weakened. Therefore, the friction of tissues can be studied by adjusting the hydrogen bonding interactions between moisture and fibers.

Based on this, the present application provides a tissue containing sodium hyaluronate, the average fiber length of the tissue is 0.7-1.4mm, the water content in the tissue is 8-20wt%, and the water activity A of the tissue conforms to the following formula:

$A < -13.78*M^2 + 4.89*M + 0.3$, wherein M is the water content in the tissue.

wherein M is the water content in the tissue.

For example, the average fiber length of the tissue can be 0.7mm, 0.8mm, 0.9mm, 1.0mm, 1.1mm, 1.2mm, 1.3mm, 1.4mm or any range therebetween.

The water content in the tissue can be 8wt%, 9wt%, 10wt%, 11wt%, 12wt%, 13wt%, 14wt%, 15wt%, 16wt%, 17wt%, 18wt%, 19wt%, 20wt% or any range therebetween.

The present application controls the average fiber length of the tissue within the above range, so that the formula of water activity and water content of the tissue satisfies the relationship of the above formula, and can reduce the friction in the tissue, that is, the tissue has a lower friction coefficient.

In the present application, the average fiber length refers to taking the arithmetic average of the fiber lengths, that is, using equipment to automatically measure 5,000 fiber lengths, and then taking the arithmetic average. The fiber length is a fiber length that can be determined by methods known to those skilled in the art. The fiber length is, for example, measured according to the method specified in International Standard ISO 16065-2.

In one embodiment, the tissue contains sodium hyaluronate and a water activity regulator. Preferably, based on 1 part by weight of sodium hyaluronate, the water activity regulator is 40-1600 parts by weight.

For example, based on 1 part by weight of sodium hyaluronate, the water activity regulator can be 40 parts by weight, 100 parts by weight, 130 parts by weight, 110 parts by weight, 250 parts by weight, 500 parts by weight, 550 parts by weight, 650 parts by weight, 700 parts by weight, 750 parts by weight, 800 parts by weight, 850 parts by weight, 900 parts by weight, 1000 parts by weight, 1300 parts by weight, 1600 parts by weight or any range therebetween.

In one embodiment, the molecular weight of the sodium hyaluronate is 100-2,500 kDa.

For example, the molecular weight of the sodium hyaluronate may be 100 kDa, 200 kDa, 300 kDa, 500 kDa, 1,000 kDa, 1,500 kDa, 2,000 kDa, 2,500 kDa or any range therebetween.

In one embodiment, the water activity regulator is a substance containing a hydroxyl group, a carboxyl group or an aldehyde group, preferably glycerol, sucrose, glucose, fructose, polyethylene glycol, butylene glycol, pentylene glycol, mannitol, sorbitol, polysorbate, or combinations thereof.

For example, the water activity regulator may be a combination of glycerin and butylene glycol or a combination of glycerin, sucrose and butylene glycol or a combination of glycerol, butylene glycol and pentylene glycol or a combination of sucrose, butylene glycol and polyethylene glycol.

The present application does not impose any restrictions on the mass ratio of various substances in the above combination and the molecular weight of polyethylene glycol, which can be routinely selected as needed. For example, when the water activity regulator is a combination of glycerin and butylene glycol, the mass ratio of the two ($m_{glycerol}: m_{butylene\ glycol}$) can be 8:2; when the water activity regulator is a combination of glycerol and butylene glycol, the mass ratio of the two ($m_{glycerol}: m_{butylene\ glycol}$) can be 7:3; when the water activity regulator is a combination of glycerol, sucrose and butylene glycol, the mass ratio of the three ($m_{glycerol}: m_{sucrose}: m_{butylene\ glycol}$) can be 7:2:1; when the water activity regulator is a combination of glycerol, butylene glycol and pentylene glycol, the mass ratio of the three ($m_{glycerol}: m_{butylene\ glycol}: m_{pentylene\ glycol}$) can be 8:1:1; when the water activity the regulator is a combination of glycerol, butylene glycol and pentylene glycol, the mass ratio of the three ($m_{glycerol}: m_{butylene\ glycol}: m_{pentylene\ glycol}$) can be 6:2:2; when the water activity regulator is a combination of sucrose, butylene glycol and polyethylene glycol (the molecular weight is 2,000Da), the mass ratio of the three ($m_{glycerol}: m_{butylene\ glycol}: m_{polyethylene\ glycol}$) can be 8:1.5:0.5.

In one embodiment, the content of the sodium hyaluronate in the tissue is 40-5700 ppm.

The content described here refers to the total content in the tissue, which is measured through the following Examples.

For example, the content of the sodium hyaluronate in the tissue can be 40ppm, 80ppm, 100ppm, 500ppm, 1000ppm, 1500ppm, 2000ppm, 2500ppm, 3000ppm, 3500ppm, 4000ppm, 4500ppm, 5000ppm, 5500ppm, 5700ppm, or any range therebetween.

In one embodiment, the cellulose content of the tissue is no less than 50% of the total mass of the tissue.

For example, the cellulose content of the tissue may be 50%, 60%, 70%, etc. of the total mass of the tissue, or any range therebetween.

In one embodiment, the tissue further contains essential oils or essences.

In one embodiment, the tissue further contains skin care ingredients, and the skin care ingredients are skin care ingredients other than sodium hyaluronate. The skin care ingredients are well known to those skilled in the art and can be routinely selected as needed.

In one embodiment, the grammage of the tissue is 10-200 gsm.

For this grammage, setting it within this range will provide a better feel. The grammage is the grammage known in the art for household paper. Tissues below 10gsm will have certain problems with the strength of the paper, while tissues above 200gsm will have very hard paper and will not be used for household paper.

For example, the grammage of the tissue can be 10gsm, 20gsm, 50gsm, 100gsm, 150gsm, 200gsm, or any range therebetween.

In one embodiment, the friction coefficient of the tissue is below 0.82, for example, it can be below 0.73, below 0.71, below 0.70, below 0.69, below 0.68, below 0.66, below 0.65, below 0.64, below 0.63, etc.

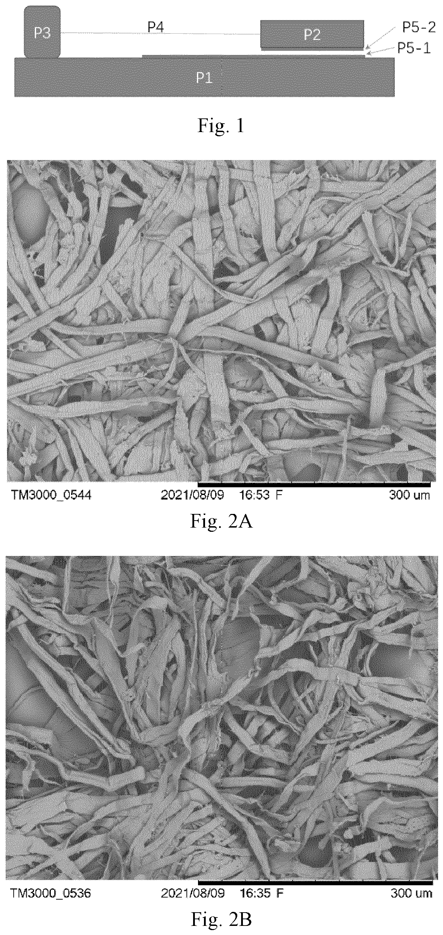

The friction coefficient is measured by using the method described in Fig. 1. P5-1 and P5-2 in the Fig. refer to the tissues obtained in the same Example or Comparative Example.

In one embodiment, the tissue may have an embossed or perforated design.

In one embodiment, the tissue may be white or colored.

In one embodiment, the average fiber length of the tissue is 0.7-1.4mm, the water content in the tissue is 8-20wt%, and the water activity A of the tissue conforms to the following formula:

$$A < -13.78 * M^2 + 4.89 * M + 0.3,$$

wherein M is the water content in the tissue. The tissue contains sodium hyaluronate and a water activity regulator. Preferably, based on 1 part by weight of sodium hyaluronate, the water activity regulator is 40-1600 parts by weight.

In one embodiment, the average fiber length of the tissue is 0.7-1.4mm, the water content in the tissue is 8-20wt%, and the water activity A of the tissue conforms to the following formula:

$$A < -13.78 * M^2 + 4.89 * M + 0.3,$$

wherein M is the water content in the tissue. The tissue contains sodium hyaluronate and a water activity regulator. Preferably, based on 1 part by weight of sodium hyaluronate, the water activity regulator is 40-1600 parts by weight, and the molecular weight of the sodium hyaluronate is 100-2,500 kDa.

In one embodiment, the average fiber length of the tissue is 0.7-1.4mm, the water content in the tissue is 8-20wt%, and the water activity A of the tissue conforms to the following formula:

$$A < -13.78 * M^2 + 4.89 * M + 0.3,$$

wherein M is the water content in the tissue. The tissue contains sodium hyaluronate and a water activity regulator. Preferably, based on 1 part by weight of sodium hyaluronate, the water activity regulator is 40-1600 parts by weight, the molecular weight of the sodium hyaluronate is 100-2,500 kDa, the water activity regulator is a substance containing a hydroxyl group, a carboxyl group or an aldehyde group, preferably glycerol, sucrose, glucose, fructose, polyethylene glycol, butylene glycol, pentylene glycol, mannitol, sorbitol, polysorbate, or combinations thereof.

In one embodiment, the average fiber length of the tissue is 0.7-1.4mm, the water content in the tissue is 8-20wt%, and the water activity A of the tissue conforms to the following formula:

$$A < -13.78 * M^2 + 4.89 * M + 0.3,$$

wherein M is the water content in the tissue. The tissue contains sodium hyaluronate and a water activity regulator. Preferably, based on 1 part by weight of sodium hyaluronate, the water activity regulator is 40-1600 parts by weight, the molecular weight of the sodium hyaluronate is 100-2,500 kDa, the water activity regulator is a substance containing a hydroxyl group, a carboxyl group or an aldehyde group, preferably glycerin, sucrose, glucose, fructose, polyethylene glycol, butylene glycol, pentylene glycol, mannitol, sorbitol, polysorbate, or a combination thereof, and the content of the sodium hyaluronate in the tissue is 40-5700ppm;

> preferably, the cellulose content of the tissue is not less than 50% of the total mass of the tissue;
> preferably, the tissue further contains essential oils or essences;
> preferably, the tissue further contains skin care ingredients;
> preferably, the grammage of the tissue is 10-200gsm.

By containing sodium hyaluronate and a water activity regulator in the tissue, the present application can adjust the friction of tissues with high-water-content, so that the friction coefficient of the tissue is below 8.2.

The present application provides a tissue finishing liquid, wherein the finishing liquid comprises hyaluronic acid and a water activity regulator; preferably, based on 1 part by weight of sodium hyaluronate, the water activity regulator is 40-1600 parts by weight;

preferably, the molecular weight of the sodium hyaluronate is 100-2,500 kDa;
preferably, the water activity regulator is a substance containing a hydroxyl group, a carboxyl group or an aldehyde group, preferably glycerol, sucrose, glucose, fructose, polyethylene glycol, butylene glycol, pentylene glycol, mannitol, sorbitol, polysorbate, or combinations thereof.

The present application provides a method for preparing tissues, wherein the issue is obtained by spraying a solution containing hyaluronic acid and a water activity regulator on a tissue base material.

The tissue base material can be any commercially available base material, such as the following tissue base material.

The tissue base material contains: about 80-95wt% cellulose fiber, about 3-7wt% moisture, and about 0.5-3wt% wet strength agent. Among them, the cellulose fiber is softwood pulp, broadleaf pulp and recycled pulp mixed in a certain proportion.

The softwood pulp refers to cellulose fibers obtained by physical and chemical purification of coniferous trees such as larch, Korean pine, spruce etc.

The broadleaf pulp refers to the cellulose fibers obtained by physical and chemical purification of broadleaf trees such as poplar, basswood, eucalyptus, maple, birch etc.

The recycled pulp refers to the cellulose fibers obtained by purifying the recycled paper products after physically and chemically removing impurities.

The wet strength agent is a polyvinyl acetate substance. Under the high temperature conditions of paper drying, this substance can undergo a cross-linking reaction, thereby improving the wet strength of the tissue.

Among them, the fiber length of softwood pulp is about 2 to 3mm, the fiber length of broadleaf pulp is about 0.4 to 1.2mm, and the fiber length of recycled pulp is about 0.2 to 0.4mm.

In the example, the chemical composition of the tissue base material is the same, but the average fiber length is different, which is obtained by different slurry ratios. In the example, a total of 3 different slurry ratios are selected, as follows:

Case-1~23 and 30~37 adopt the same slurry ratio, softwood pulp (wt): broadleaf pulp (wt): recycled pulp (wt) = 1:4:0. The difference in the average fiber length in the test is due to the distribution of fiber lengths in the slurry, not to changes in the ratio.
Case-24~26 adopt the same slurry ratio, softwood pulp (wt): broadleaf pulp (wt): recycled pulp (wt) = 3:1:0. The difference in the average fiber length in the test is due to the distribution of fiber length in the slurry, not to changes in the ratio.
Case-27~29 adopt the same slurry ratio, softwood pulp (wt): broadleaf pulp (wt): recycled pulp (wt) = 0.3:1:4. The difference in the average fiber length in the test is due to the distribution of fiber lengths in the slurry, not to changes in the ratio.

The solution containing hyaluronic acid and a water activity regulator refers to a solution obtained by dissolving hyaluronic acid and the water activity regulator in water.

In one embodiment, the molecular weight of the sodium hyaluronate is 100-2,500 kDa.

In one embodiment, the content of sodium hyaluronate in the tissue is 40-5700 ppm.

In one embodiment, the water activity regulator is a substance containing a hydroxyl group, a carboxyl group or an aldehyde group, preferably glycerol, sucrose, glucose, fructose, polyethylene glycol, butylene glycol, pentylene glycol, mannitol, sorbitol, polysorbate or combinations thereof.

In one embodiment, based on 1 part by weight of sodium hyaluronate, the water activity regulator is 40-1600 parts by weight.

In a solution containing sodium hyaluronate and a water activity regulator, it means that when 1 part by weight of sodium hyaluronate is added, 40-1600 parts by weight of the water activity regulator need to be added.

The average fiber length of the tissues obtained by the present application is 0.7-1.4mm, the water content in the tissues is 8-20%, and the water activity A of the tissues conforms to the following formula:

$$A < -13.78 * M^2 + 4.89 * M + 0.3,$$

wherein M is the water content in the tissue.

The friction of the obtained tissue is small, and the friction coefficient of the tissue is below 0.82. The obtained tissue contains hyaluronic acid and a water activity regulator. By adjusting the relationship between the water activity and moisture in the tissue, the friction of tissues can be significantly reduced.

## EXAMPLES

The present application provides a general and/or specific description of the materials and test methods used in the test. In the following examples, if there is no other special explanation, % means wt%, that is, weight percentage. If the manufacturer of the reagents or instruments used is not indicated, they are all conventional reagent products that can be purchased commercially. Among them, sodium hyaluronate was purchased from the Jinan factory of Bloomage Biotechnology. In the example, the chemical composition of the tissue base material is the same, but the average fiber length is different, which is obtained by different slurry ratios. The fiber base material contains about 91% cellulose fibers, about 7% moisture and about 2% wet strength agent. The softwood pulp is made from Southern yellow pine, broadleaf pulp is made from eucalyptus; and the wet strength agent is VINNAPAS®-192 from WACKER CHEMICAL.

## Example 1

Sodium hyaluronate of different molecular weights and water activity regulators were dissolved in deionized water to obtain a solution, and the solution was coated on a tissue base material to obtain a tissue, wherein the contents of sodium hyaluronate and the water activity regulators in the solution were shown in cases 9-23 and 30-37 in Table 1, wherein, the tissue base materials use the same slurry ratio, softwood pulp (wt): broadleaf pulp (wt): recycled pulp (wt) = 1: 4:0.

## Comparative Example 1

Sodium hyaluronate of different molecular weights and water activity regulators were dissolved in deionized water to obtain a solution, and the solution was coated on a tissue base material to obtain a tissue, wherein the contents of sodium hyaluronate and the water activity regulators in the solution were shown in cases 1-8 and 24-29 in Table 1, wherein, the tissue base materials of case 1-8 use the same slurry ratio, softwood pulp (wt): broadleaf pulp (wt): recycled pulp (wt) = 1: 4:0.,

The tissue base materials of case 24-26 use the same slurry ratio, softwood pulp (wt): broadleaf pulp (wt): recycled pulp (wt) = 3:1:0;

The tissue base materials of case 27-29 use the same slurry ratio, softwood pulp (wt): broadleaf pulp (wt): recycled pulp (wt) = 0.3:1:4.

Table 1 Contents of sodium hyaluronate and a water activity regulator in solutions in Example 1 and Comparative Example 1

| Case | The content (%) and molecular weight of hyaluronic acid (HA) | The content of water activity regulators |
|---|---|---|
| 1 | 0.1% HA (300 kDa) | - |
| 2 | 0.1% HA (300 kDa) | - |
| 3 | 0.1% HA (300 kDa) | - |
| 4 | 0.1% HA (300 kDa) | |
| 5 | 0.1% HA (300 kDa) | 50% water activity regulator (the water activity regulator was glycerol and butylene glycol, and the mass ratio was 9:1) |
| 6 | 0.1% HA (300 kDa) | 50% water activity regulator (the water activity regulator was glycerol and butylene glycol, and the mass ratio was 9:1) |
| 7 | 0.1% HA (300 kDa) | 50% water activity regulator (the water activity regulator was glycerol and butylene glycol, and the mass ratio was 9:1) |
| 8 | 0.1% HA (300 kDa) | 50% water activity regulator (the water activity regulator was glycerol and butylene glycol, and the mass ratio was 9:1) |
| 9 | 0.1% HA (300 kDa) | 65% water activity regulator (the water activity regulator was glycerol and butylene glycol, and the mass ratio was 8:2) |

(continued)

| Case | The content (%) and molecular weight of hyaluronic acid (HA) | The content of water activity regulators |
|---|---|---|
| 10 | 0.1% HA (300 kDa) | 65% water activity regulator (the water activity regulator was glycerol and butylene glycol, and the mass ratio was 8:2) |
| 11 | 0.1% HA (300 kDa) | 65% water activity regulator (water activity regulator was glycerol and |
|  |  | butylene glycol, and the mass ratio was 8:2) |
| 12 | 0.1% HA (300 kDa), | 65% water activity regulator (the water activity regulator was glycerol and butylene glycol, and the mass ratio was 8:2) |
| 13 | 0.1% HA (300 kDa) | 65% water activity regulator (the water activity regulator was glycerol and butylene glycol, and the mass ratio was 8:2) |
| 14 | 0.1% HA (300 kDa) | 70% water activity regulator (the water activity regulator was glycerol, sucrose, and butylene glycol, and the mass ratio was 7:2:1) |
| 15 | 0.1% HA (300 kDa) | 70% water activity regulator (the water activity regulator was glycerol, sucrose, and butylene glycol, and the mass ratio was 7:2:1) |
| 16 | 0.1% HA (300 kDa) | 70% water activity regulator (the water activity regulator was glycerol, sucrose, and butylene glycol, and the mass ratio was 7:2:1) |
| 17 | 0.1% HA (300 kDa) | 70% water activity regulator (the water activity regulator was glycerol, sucrose, and butylene glycol, and the mass ratio was 7:2:1) |
| 18 | 0.1% HA (300 kDa) | 70% water activity regulator (the water activity regulator was glycerol, sucrose, and butylene glycol, and the mass ratio was 7:2:1) |
| 19 | 0.1% HA (300 kDa) | 80% water activity regulator (the water activity regulator was glycerol, butylene glycol, and pentylene glycol, and the mass ratio was 8:1:1) |
| 20 | 0.1% HA (300 kDa) | 80% water activity regulator (the water activity regulator was glycerol, butylene glycol, and pentylene glycol, and the mass ratio was 8:1:1) |
| 21 | 0.1% HA (300 kDa) | 80% water activity regulator (the water activity regulator was glycerol, butylene glycol, and pentylene glycol, and the mass ratio was 8:1:1) |
| 22 | 0.1% HA (300 kDa) | 80% water activity regulator (the water activity regulator was glycerol, butylene glycol, and pentylene glycol, and the mass ratio was 8:1:1) |
| 23 | 0.1% HA (300 kDa) | 80% water activity regulator (the water activity regulator was glycerol, butylene glycol, and pentylene glycol, and the mass ratio was 8:1:1) |
| 24 | 0.1% HA (300 kDa) | 70% water activity regulator (the water activity regulator was glycerol, sucrose, and butylene glycol, and the mass ratio was 7:2:1) |
| 25 | 0.1% HA (300 kDa) | 70% water activity regulator (the water activity regulator was glycerol, sucrose, and butylene glycol, and the mass ratio was 7:2:1) |

(continued)

| Case | The content (%) and molecular weight of hyaluronic acid (HA) | The content of water activity regulators |
|---|---|---|
| 26 | 0.1% HA (300 kDa) | 70% water activity regulator (the water activity regulator was glycerol, sucrose, and butylene glycol, and the mass ratio was 7:2:1) |
| 27 | 0.1% HA (300 kDa), | 70% water activity regulator (the water activity regulator was glycerol, sucrose, and butylene glycol, and the mass ratio was 7:2:1) |
| 28 | 0.1% HA (300 kDa) | 70% water activity regulator (the water activity regulator was glycerol, sucrose, and butylene glycol, and the mass ratio was 7:2:1) |
| 29 | 0.1% HA (300 kDa) | 70% water activity regulator (the water activity regulator was glycerol, sucrose, and butylene glycol, and the mass ratio was 7:2:1) |
| 30 | 0.5% HA (300 kDa), | 65% water activity regulator (water activity regulator was glycerol, butylene glycol, the mass ratio was 7:3 ) |
| 31 | 0.5% HA (300 kDa) | 65% water activity regulator (the water activity regulator was glycerol and butylene glycol, and the mass ratio was 7:3) |
| 32 | 1.5% HA (300 kDa) | 65% water activity regulator (the water activity regulator was glycerol, butylene glycol, and pentylene glycol, and the mass ratio was 6:2:2) |
| 33 | 1.5% HA (300 kDa) | 65% water activity regulator (the water activity regulator was glycerol, butylene glycol, and pentylene glycol, and the mass ratio was 6:2:2) |
| 34 | 0.5% HA (2,000 kDa) | 65% water activity regulator (the water activity regulator was glycerol and butylene glycol, and the mass ratio was 7:3) |
| 35 | 0.5% HA (2,000 kDa) | 65% water activity regulator (the water activity regulator was glycerol and butylene glycol, and the mass ratio was 7:3) |
| 36 | 0.5% HA (300 kDa) | 55% water activity regulator (the water activity regulator was sucrose, butylene glycol, and PEG, the molecular weight of PEG was 2000, and the mass ratio was 8:1.5:0.5) |
| 37 | 0.05% HA (2,000 kDa) | 80% water activity regulator (the water activity regulator was glycerol, butylene glycol, and pentylene glycol, and the mass ratio was 8:1:1) |

**Experimental Example 1: Determination of HA and water content in tissues**

Determination of HA: Determination was carried out using the method described in CN109298112A, and the results were shown in Table 2.

Determination of water content: the weight of a tissue prepared in Example 1 and Comparative Example 1 was accurately weighed by an analytical balance, and recorded as W1. If the tissue was composed of several low-grammage tissue base materials through lamination, the total weight of the lamination paper was weighed, and recorded as W1.

The tissue was placed in an oven at 105 degrees Celsius (+/-2 degrees Celsius) and dried for 30 minutes (+/- 1 minute), it was taken out quickly, placed on the analytical balance within 20 seconds, and read the weight, recorded as W2. Since there will be a certain amount of water vapor in the air, the dried tissues will continue to absorb moisture in the environment, so the weight needs to be measured and the data needs to be recorded within 20 seconds after being taken out of the oven.

Water content M=(W1-W2)/W1, and the results were shown in Table 2:

Table 2 HA and water contents in tissues

| Case | HA content (ppm) | Water content (%) |
|------|------------------|-------------------|
| 1 | 50 | 8.5% |
| 2 | 60 | 9.4% |
| 3 | 110 | 14.9% |
| 4 | 250 | 27.1% |
| 5 | 60 | 9.1% |
| 6 | 130 | 13.6% |
| 7 | 190 | 15.3% |
| 8 | 320 | 21.0% |
| 9 | 50 | 8.3% |
| 10 | 150 | 11.5% |
| 11 | 300 | 15.1% |
| 12 | 390 | 18.6% |
| 13 | 430 | 19.9% |
| 14 | 80 | 8.7% |
| 15 | 120 | 9.7% |
| 16 | 200 | 11.5% |
| 17 | 310 | 14.4% |
| 18 | 400 | 16.5% |
| 19 | 90 | 8.7% |
| 20 | 170 | 9.3% |
| 21 | 220 | 9.9% |
| 22 | 270 | 10.6% |
| 23 | 390 | 12.2% |
| 24 | 120 | 9.5% |
| 25 | 270 | 13.3% |
| 26 | 380 | 15.9% |
| 27 | 140 | 10.2% |
| 28 | 300 | 14.1% |
| 29 | 370 | 15.8% |
| 30 | 280 | 8.7% |
| 31 | 1680 | 16.8% |
| 32 | 1380 | 9.3% |
| 33 | 5630 | 17.4% |
| 34 | 430 | 9.7% |
| 35 | 1790 | 17.1% |
| 36 | 520 | 11.4% |
| 37 | 40 | 8.2% |

**Experimental Example 2: Determination of water activity of tissues**

"Swiss ROTRONIC Hygrolab-C1-SET Desktop Water Activity Meter" or equivalent equipment was used.

One tissue from Example 1 and Comparative Example 1 were taken and placed in the sample pool for water activity detection. Among them, the weight of the tissues must not be less than 1.0 grams. If the weight of the tissues was less than 1.0 grams, several tissues should be stacked so that the total weight of the tissues was greater than 1.0 grams.

Then, the sample pool was sealed with a test probe and left to stand in an environment of 25 degrees Celsius (+/-2 degrees Celsius) for 1 hour. When the water activity value reached a stable value, the water activity was recorded. Each sample was tested 3 times and the average value was taken, the results were shown in Table 3.

Table 3 Water activity of tissues

| Case | Water activity |
| --- | --- |
| 1 | 0.799 |
| 2 | 0.937 |
| 3 | 0.976 |
| 4 | 0.994 |
| 5 | 0.721 |
| 6 | 0.893 |
| 7 | 0.913 |
| 8 | 0.933 |
| 9 | 0.573 |
| 10 | 0.653 |
| 11 | 0.673 |
| 12 | 0.687 |
| 13 | 0.691 |
| 14 | 0.507 |
| 15 | 0.552 |
| 16 | 0.578 |
| 17 | 0.588 |
| 18 | 0.593 |
| 19 | 0.427 |
| 20 | 0.455 |
| 21 | 0.487 |
| 22 | 0.498 |
| 23 | 0.501 |
| 24 | 0.566 |
| 25 | 0.581 |
| 26 | 0.590 |
| 27 | 0.577 |
| 28 | 0.598 |
| 29 | 0.621 |
| 30 | 0.579 |

(continued)

| Case | Water activity |
|------|----------------|
| 31 | 0.674 |
| 32 | 0.581 |
| 33 | 0.691 |
| 34 | 0.597 |
| 35 | 0.689 |
| 36 | 0.631 |
| 37 | 0.413 |

The water content of the tissue of Example 1 obtained in Experimental Example 2 and the water activity of the tissue of Example 1 obtained in the Experimental Example 2 were fitted using the least squares method, and the following formula was obtained: $A < -13.78 * M^2 + 4.89 * M + 0.3$, wherein M was the water content of the tissue in Example 1.

**Experimental Example 3: Determination of friction coefficient**

First, in order to determine the acceptable threshold of tissue friction, friction tests and consumer blind tests were conducted using different paper products sold on the market. In the consumer blind test experiment, the consumers were 30 female white-collar workers in Beijing aged 19 to 40. The four samples Ctrl, B 1L, B1M, and B 1H were touched by each subject, and scores were given by them according to the softness. 1 was the worst and 10 was the best. The results were shown in Table 5. Among them, the information of the four samples was shown in Table 4.

Table 4 Information of 4 samples

| product code | Product Details |
|--------------|-----------------|
| Ctrl | unbleached, natural color, bamboo fiber extractable tissue of BASE series of BABO® |
| B1L | unscented, wettable facial tissue of Face series of C & S® |
| B1M | unscented, extractable moisturizing facial tissue of Doraemon series of NEPIA® |
| B1H | extractable lotion tissue of Premium series of NEPIA® |

Table 5 Acceptance results for four samples

|  | product code | | | |
|---|---|---|---|---|
|  | Ctrl | B1L | B1M | B1H |
| frictional coefficient | 1.12 | 0.82 | 0.74 | 0.66 |
| Panel-1 | 1 | 3 | 7 | 9 |
| Panel-2 | 1 | 4 | 7 | 9 |
| Panel-3 | 1 | 3 | 7 | 9 |
| Panel-4 | 1 | 2 | 7 | 10 |
| Panel-5 | 1 | 3 | 7 | 8 |
| Panel-6 | 1 | 3 | 7 | 9 |
| Panel-7 | 2 | 2 | 8 | 10 |
| Panel-8 | 1 | 3 | 7 | 9 |
| Panel-9 | 2 | 3 | 6 | 9 |
| Panel-10 | 2 | 4 | 7 | 9 |

(continued)

| | product code | | | |
|---|---|---|---|---|
| | Ctrl | B1L | B1M | B1H |
| Panel-11 | 1 | 3 | 6 | 9 |
| Panel-12 | 1 | 3 | 7 | 10 |
| Panel-13 | 1 | 3 | 8 | 10 |
| Panel-14 | 1 | 2 | 8 | 9 |
| Panel-15 | 1 | 2 | 8 | 9 |
| Panel-16 | 1 | 3 | 7 | 8 |
| Panel-17 | 1 | 4 | 7 | 9 |
| Panel-18 | 1 | 3 | 7 | 9 |
| Panel-19 | 2 | 4 | 7 | 10 |
| Panel-20 | 2 | 4 | 6 | 9 |
| Panel-21 | 2 | 4 | 7 | 10 |
| Panel-22 | 1 | 3 | 8 | 10 |
| Panel-23 | 1 | 3 | 7 | 9 |
| Panel-24 | 2 | 4 | 7 | 9 |
| Panel-25 | 1 | 3 | 6 | 10 |
| Panel-26 | 2 | 3 | 7 | 9 |
| Panel-27 | 1 | 3 | 7 | 9 |
| Panel-28 | 1 | 3 | 7 | 9 |
| Panel-29 | 1 | 3 | 8 | 9 |
| Panel-30 | 1 | 3 | 7 | 9 |

As can be seen from the table above, when the friction coefficient was 0.82, it was the acceptable threshold for tissue friction.

Therefore, the friction coefficient of the tissues of Example 1 and Comparative Example 1 were measured, and the measurement method was as follows:

The friction was tested using PARAM® MXD-02 friction coefficient meter or equivalent equipment. The specific test method was shown in Fig. 1. The friction detection equipment consisted of a horizontal platform P1, a movable pressure slider P2, a motor P3 with a tension sensing device and a rigid traction rope P4. The tissues P5-1 and P5-2 (the tissues of Example 1 and Comparative Example 1) to be tested were fixed on the surfaces of P1 and P2 respectively.

During the test, the movable slider P2 moved horizontally and uniformly toward P3 at a speed of 150mm/min under the traction of the rigid traction rope P4. The mass of the movable slider P2 (MP) was 100g, and the area of the movable slider P2 (SP) was 64cm$^2$. The process of moving at a constant speed ensures that all test surfaces of P5-2 are within the area of P5-1.

In uniform motion, the average friction was recorded as FA, then the friction coefficient CoF=FA/(MP*g), wherein g=9.8m/s$^2$, and the results were shown in Table 6.

Table 6 Friction coefficient of tissues

| Case | Friction coefficient |
|---|---|
| 1 | 0.97 |
| 2 | 0.97 |
| 3 | 0.95 |
| 4 | 0.94 |

(continued)

| Case | Friction coefficient |
|------|---------------------|
| 5 | 0.9 |
| 6 | 0.89 |
| 7 | 0.89 |
| 8 | 0.91 |
| 9 | 0.71 |
| 10 | 0.7 |
| 11 | 0.69 |
| 12 | 0.7 |
| 13 | 0.71 |
| 14 | 0.69 |
| 15 | 0.67 |
| 16 | 0.66 |
| 17 | 0.69 |
| 18 | 0.69 |
| 19 | 0.66 |
| 20 | 0.65 |
| 21 | 0.63 |
| 22 | 0.63 |
| 23 | 0.69 |
| 24 | 0.87 |
| 25 | 0.85 |
| 26 | 0.82 |
| 27 | 0.95 |
| 28 | 0.93 |
| 29 | 0.93 |
| 30 | 0.69 |
| 31 | 0.68 |
| 32 | 0.68 |
| 33 | 0.69 |
| 34 | 0.69 |
| 35 | 0.68 |
| 36 | 0.73 |
| 37 | 0.64 |

**Experimental Example 4: Determination of tissue fiber length**

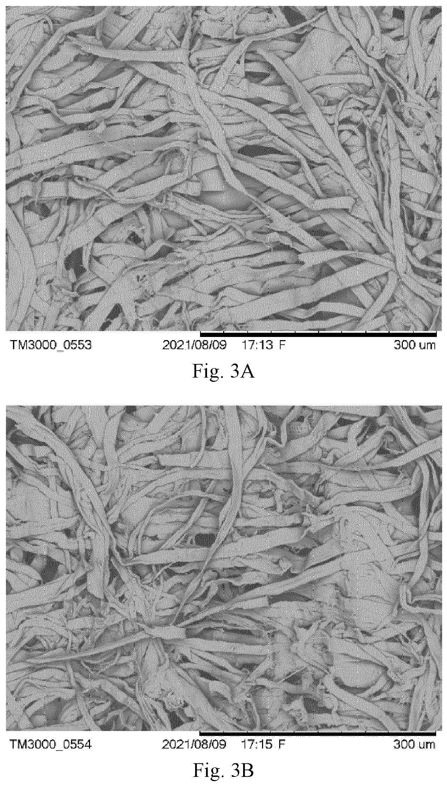

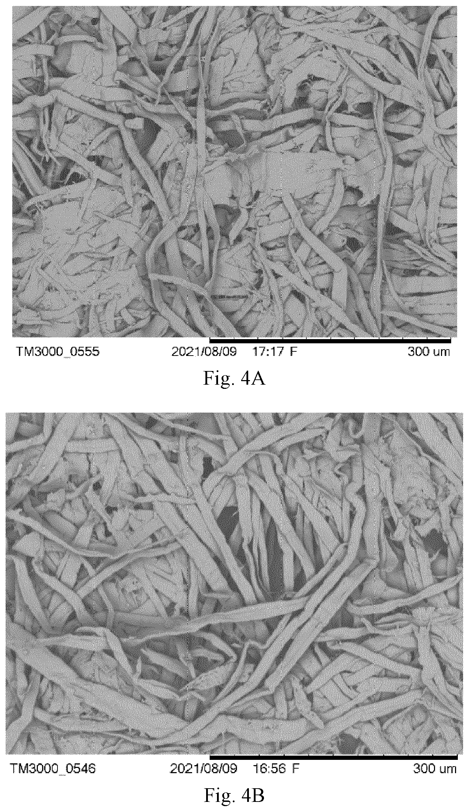

The test was carried out in accordance with International Standard ISO 16065-2, wherein 5000 points were tested for each sample to analyze the fiber length. The results were shown in Table 7. Among them, the tissues in case 1 were taken pictures before and after rubbing using a transmission electron microscope (Hitachi The high-tech AeroSurf1500 desktop scanning electron microscope), and the TEM of the fiber arrangement was shown in Fig. 2A and Fig. 2B. Similarly, the fiber arrangement of case 9 and case 13 before and after rubbing were shown in Fig. 3A and Fig. 3B, and Fig. 4A and Fig. 4B.

**Table 7 fiber length**

| Case | The fiber length of tissues $D_{90}$ |
|------|----------------------------------------|
| 1 | 1.13 |
| 2 | 0.99 |
| 3 | 0.73 |
| 4 | 0.84 |
| 5 | 0.88 |
| 6 | 1.34 |
| 7 | 1.32 |
| 8 | 1.11 |
| 9 | 1.16 |
| 10 | 0.98 |
| 11 | 0.74 |
| 12 | 1.34 |
| 13 | 1.15 |
| 14 | 0.97 |
| 15 | 0.82 |
| 16 | 1.21 |
| 17 | 0.75 |
| 18 | 1.38 |
| 19 | 1.13 |
| 20 | 0.91 |
| 21 | 0.99 |
| 22 | 0.83 |
| 23 | 0.75 |
| 24 | 1.67 |
| 25 | 1.94 |
| 26 | 1.76 |
| 27 | 0.62 |
| 28 | 0.53 |
| 29 | 0.55 |
| 30 | 1.27 |
| 31 | 1.18 |
| 32 | 0.79 |
| 33 | 0.75 |
| 34 | 0.77 |
| 35 | 0.93 |
| 36 | 1.13 |

(continued)

| Case | The fiber length of tissues $D_{90}$ |
|---|---|
| 37 | 0.89 |

It can be seen from Figs. 3A and 3B and Figs. 4A and 4B that after rubbing at the interface of case 9 and case13 tissues, the fibers showed a flat spreading state, while from Fig. 2A and Fig. 2B, the detachment phenomenon of fiber cohering was shown, indicating that by adjusting the water activity, the cohesion between fibers was not affected, indicating that the friction of the tissue of the present application was reduced. In addition, from the data of the friction measured in the experimental examples, it can be seen that the friction coefficient of the tissue after rubbing in case 2 was 0.97, which was higher than the friction coefficient of the tissue base material without sodium hyaluronate coating, which was 0.91.

It can be seen from Table 7 that the average fiber length of the tissues of the present application was 0.7-1.4mm, and the difference in the average fiber length in the test was due to the distribution of fiber lengths in the slurry, not to the change in the ratio.

To sum up, when the average fiber length of the tissue of the present application was 0.7-1.4 mm, by adjusting the water content and water activity in the tissue, the friction coefficient of the tissue can be reduced, so that the friction coefficient of the tissue was below 0.82.

The above are only preferred Examples of the present application, and are not intended to limit the present application in other forms. Any skilled person familiar with the art may make changes or modifications to equivalent Examples using the technical contents disclosed above. However, any simple modifications, equivalent changes and modifications made to the above Examples based on the technical essence of the present application without departing from the content of the technical solution of the present application still fall within the protection scope of the technical solution of the present application.

**Claims**

1.   A tissue containing sodium hyaluronate with an average fiber length of 0.7-1.4mm and a water content of 8-20wt%, wherein the water activity A of the tissue conforms to the following formula:

$$A < -13.78*M^2 + 4.89*M + 0.3,$$

wherein M is the water content in the tissue.

2.   The tissue according to claim 1, wherein the tissue contains sodium hyaluronate and a water activity regulator, preferably, based on 1 part by weight of sodium hyaluronate, the water activity regulator is 40-1600 parts by weight.

3.   The tissue according to claim 2, wherein the molecular weight of the sodium hyaluronate is 100-2,500 kDa.

4.   The tissue according to claim 2 or 3, wherein the water activity regulator is a substance containing a hydroxyl group, a carboxyl group or an aldehyde group, preferably glycerol, sucrose, glucose, fructose, polyethylene glycol, butylene glycol, pentylene glycol, mannitol, sorbitol, polysorbate, or combinations thereof.

5.   The tissue according to any one of claims 1 to 4, wherein the content of the sodium hyaluronate in the tissue is 40-5700 ppm.

6.   The tissue according to any one of claims 1 to 5, wherein the cellulose content of the tissue is not less than 50% of the total mass of the tissue.

7.   The tissue according to any one of claims 1 to 6, wherein the tissue further contains essential oils or essences;

preferably, the tissue further contains skin care ingredients;
Preferably, the grammage of the tissue is 10-200gsm.

8.   A tissue finishing liquid, comprising hyaluronic acid and a water activity regulator; preferably, based on 1 part by weight of sodium hyaluronate, the water activity regulator is 40-1600 parts by weight;

preferably, the molecular weight of the sodium hyaluronate is 100-2,500 kDa;
preferably, the water activity regulator is a substance containing a hydroxyl group, a carboxyl group or an aldehyde group, preferably glycerol, sucrose, glucose, fructose, polyethylene glycol, butylene glycol, pentylene glycol, mannitol, sorbitol, polysorbate, or combinations thereof.

9. A method for preparing a tissue, wherein the tissue is obtained by spraying a solution containing hyaluronic acid and a water activity regulator on a tissue base material.

10. The method according to claim 9, wherein the molecular weight of sodium hyaluronate is 100-2,500 kDa;

preferably, the content of the sodium hyaluronate in the tissue is 40-5700ppm;
preferably, the water activity regulator is a substance containing a hydroxyl group, a carboxyl group or an aldehyde group, preferably glycerol, sucrose, glucose, fructose, polyethylene glycol, butylene glycol, pentylene glycol, mannitol, sorbitol, polysorbate, or combinations thereof;
preferably, based on 1 part by weight of sodium hyaluronate, the water activity regulator is 40-1600 parts by weight.

Fig. 1

Fig. 2A

Fig. 2B

TM3000_0553      2021/08/09   17:13 F       300 um

Fig. 3A

TM3000_0554      2021/08/09   17:15 F       300 um

Fig. 3B

TM3000_0555          2021/08/09   17:17 F          300 um

Fig. 4A

TM3000_0546          2021/08/09   16:56 F          300 um

Fig. 4B

## INTERNATIONAL SEARCH REPORT

| International application No. |
|---|
| **PCT/CN2021/130870** |

**A. CLASSIFICATION OF SUBJECT MATTER**

A47K 10/16(2006.01)i

According to International Patent Classification (IPC) or to both national classification and IPC

**B. FIELDS SEARCHED**

Minimum documentation searched (classification system followed by classification symbols)

A47K

Documentation searched other than minimum documentation to the extent that such documents are included in the fields searched

Electronic data base consulted during the international search (name of data base and, where practicable, search terms used)

CNABS; CNTXT; VEN; USTXT; WOTXT; EPTXT; CNKI: 华熙海御, 华熙生物, 刘喆, 杨娟, 纸巾, 棉柔巾, 化妆棉, 摩擦, 粗糙, 韧性, 韧度, 掉屑, 透明质酸, 玻璃酸, 玻尿酸, 分子量, 含水量, 水分含量, 水含量, 水活度, 水分活度, 水活性, 调节剂, 羟基, 羧基, 醛基, 甘油, 蔗糖, 葡萄糖, 果糖, 聚乙二醇, 丁二醇, 戊二醇, 甘露醇, 山梨醇, 聚山梨醇, tissue, friction, roughness, resilience, HA, DA, water activity, aw, wt

**C. DOCUMENTS CONSIDERED TO BE RELEVANT**

| Category* | Citation of document, with indication, where appropriate, of the relevant passages | Relevant to claim No. |
|---|---|---|
| X | CN 107049834 A (WUHAN CHUANGXIN OUPAI TECHNOLOGY CO., LTD.) 18 August 2017 (2017-08-18)<br>  description, paragraphs [0012]-[0014] | 8-10 |
| A | CN 110494610 A (MIYOSHI YUSHI K.K.) 22 November 2019 (2019-11-22)<br>  description, paragraphs [0027]-[0070] | 1-10 |
| A | CN 110004764 A (BLOOMAGE BIOTECHNOLOGY CORP., LTD. et al.) 12 July 2019 (2019-07-12)<br>  entire document | 1-10 |
| A | CN 109518531 A (ZHEJIANG FENGZHOU SPECIAL PAPER CO., LTD.) 26 March 2019 (2019-03-26)<br>  entire document | 1-10 |
| A | CN 111629640 A (DAIO PAPER K.K.) 04 September 2020 (2020-09-04)<br>  entire document | 1-10 |
| A | KR 101990743 B1 (Yoo Jung Hwan) 18 June 2019 (2019-06-18)<br>  entire document | 1-10 |

☐ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **09 May 2022** | **27 May 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
Information on patent family members

International application No.

**PCT/CN2021/130870**

| Patent document cited in search report | | | Publication date (day/month/year) | Patent family member(s) | | | Publication date (day/month/year) |
|---|---|---|---|---|---|---|---|
| CN | 107049834 | A | 18 August 2017 | None | | | |
| CN | 110494610 | A | 22 November 2019 | BR | 112020018599 | A2 | 29 December 2020 |
| | | | | EP | 3767030 | A1 | 20 January 2021 |
| | | | | TW | 201942448 | A | 01 November 2019 |
| | | | | US | 2021047783 | A1 | 18 February 2021 |
| | | | | AU | 2019235154 | A1 | 24 September 2020 |
| | | | | WO | 2019176644 | A1 | 19 September 2019 |
| | | | | JP | 6556282 | B1 | 07 August 2019 |
| | | | | JP | 2019157307 | A | 19 September 2019 |
| | | | | TW | 678446 | B1 | 01 December 2019 |
| | | | | CN | 110494610 | B | 28 September 2021 |
| | | | | EP | 3767030 | A4 | 15 December 2021 |
| CN | 110004764 | A | 12 July 2019 | None | | | |
| CN | 109518531 | A | 26 March 2019 | None | | | |
| CN | 111629640 | A | 04 September 2020 | EP | 3760089 | A1 | 06 January 2021 |
| | | | | KR | 20200127984 | A | 11 November 2020 |
| | | | | WO | 2019167747 | A1 | 06 September 2019 |
| | | | | US | 2020407922 | A1 | 31 December 2020 |
| | | | | JP | 2019150138 | A | 12 September 2019 |
| | | | | EP | 3760089 | A4 | 03 November 2021 |
| KR | 101990743 | B1 | 18 June 2019 | None | | | |

Form PCT/ISA/210 (patent family annex) (January 2015)